# DEMANDE DE BREVET EUROPEEN

(11) **EP 1 048 671 A1**
(43) Date de publication de la demande: **02.11.2000**
(21) Numéro de dépôt: 00401124.3
(22) Date de dépôt: 21.04.2000
(51) Int. Cl.: C07H 3/02, C13K 13/00, A61K 31/70, A61K 7/00, A23L 1/236

(54) **Procédé de préparation d'une composition de tagatose à partir de galactosone**

(30) Priorité: 27.04.1999 FR 9905310
(71) Demandeur: Roquette Frères, 62136 Lestrem (FR)
(72) Inventeur: Choque, Jean-Christophe, 59000 Lille (FR); Fleche, Guy, 59190 Hazebrouck (FR)
(74) Mandataire: Boulinguiez, Didier

(57) **Abrégé**

L'invention a pour objet un procédé de préparation d'une composition de tagatose par hydrogénation catalytique, à une température au moins égale à 40°C et à une pression au moins égale à 10 bars, d'une composition de galactosone présentant une matière sèche au moins égale à 10 %.

Le catalyseur utilisé lors du traitement d'hydrogénation peut notamment consister en du palladium sur charbon. La composition de galactosone soumise à hydrogénation peut, ou non, contenir d'autres sucres tels que de la glucosone ou du fructose.

Cette composition peut notamment être obtenue à partir d'une composition de galactose telle qu'un hydrolysat de lactose ou de lactulose, purifié ou non, ayant été soumis à l'action d'une enzyme à activité pyranose 2-oxydasique.

Le procédé revendiqué permet d'obtenir des compositions de tagatose (hydrogéné ou non) contenant également du sorbitol et, éventuellement, du mannitol.

L'ensemble de ces compositions peuvent avantageusement être utilisées dans la préparation de produits alimentaires, pharmaceutiques ou cosmétiques.

## Description

La présente invention a pour objet un nouveau procédé de préparation d'une composition de tagatose comprenant une étape d'hydrogénation catalytique d'une composition de galactosone. Elle vise également, en tant que produits nouveaux, certaines des compositions de tagatose obtenues selon ce procédé ainsi que les utilisations industrielles, en particulier alimentaires, pharmaceutiques et cosmétiques de l'ensemble des compositions obtenues selon ledit procédé.

Le tagatose ou lyxo-hexulose est un sucre de faible caloricité avantageusement applicable en tant qu'édulcorant et agent de charge comme il résulte du brevet EP 257.626. Ce produit présenterait également l'intérêt d'être un agent anti-hyperglycémique utile notamment pour le traitement du diabète comme décrit dans le brevet US 5.447.917.

Par ailleurs, le brevet EP 552.894 souligne la possibilité d'exploiter le tagatose également comme intermédiaire pour la synthèse de composés optiquement actifs ou comme additif dans les compositions détergentes ou cosmétiques.

Cependant, l'exploitation commerciale de ce sucre s'est toujours heurtée, en particulier, à l'absence d'un moyen apte à le préparer industriellement dans des conditions économiques satisfaisantes.

Aux fins de pallier ce problème, il a été préconisé différentes voies de synthèse du tagatose à partir de compositions de galactose, y compris de mélanges contenant du galactose et au moins un autre sucre comme le glucose, mélanges tels que ceux résultant de traitements d'hydrolyse du lactose purifié ou encore contenu dans des coproduits industriels comme le petit-lait déprotéinisé.

Ainsi, le brevet EP 518.874 préconise un procédé de préparation de tagatose prévoyant l'isomérisation catalytique d'une solution aqueuse de galactose, menée dans des conditions très particulières (pH, température, nature des catalyseurs) en vue de former un précipité insoluble composé essentiellement d'un complexe hydroxyde de métal - tagatose, puis la neutralisation dudit précipité par un acide approprié.

Cette technologie présente les inconvénients d'être très complexe et de rendre obligatoire un contrôle très strict des conditions de pH, de température et d'agitation du milieu réactionnel. Par ailleurs, les rendements en tagatose apparaissent limités, en tous cas inférieurs à 75 % en poids.

Il a également été décrit, dans le brevet EP 552.894 précité, la synthèse de tagatose par isomérisation enzymatique de compositions de galactose à l'aide d'une L-arabinose isomérase.

Il apparaît cependant que même dans les conditions réactionnelles optimales, et notamment en présence d'activateurs métalliques de l'enzyme, les rendements en tagatose sont au plus égaux à 45 %.

Plus récemment, il a été envisagé par S. FREIMUND et al. (J. CARBOHYDRATE CHEMISTRY, 15 (1), 115-120 (1996)) la synthèse de tagatose par une voie chimicoenzymatique en deux étapes à partir de galactose pur.

La première étape consiste à oxyder enzymatiquement le galactose par la pyranose 2-oxydase en présence de catalase en vue de préparer de la galactosone. La seconde étape consiste à hydrogéner catalytiquement la galactosone, purifiée ou non, pendant plusieurs jours en présence d'un catalyseur de type palladium sur charbon (« Pd/C ») et sous une pression d'hydrogène de 1,5 atmosphère soit environ 1,5 bar. La température réactionnelle et la richesse en palladium du catalyseur ne sont pas précisées. Il apparaît que dans les conditions décrites, la conversion de la galactosone est totale puisque les auteurs ne décèlent pas sa présence dans le milieu réactionnel final. Par contre, la sélectivité de cette réaction d'hydrogénation est relativement faible puisque, dans le meilleur des cas (galactosone purifiée), on obtient un mélange réactionnel ne contenant que 76 % en poids de tagatose avec formation concomitante et significative à la fois de galactose (16 %) et de talose (8 %).

Cette sélectivité limitée, liée à l'étape d'hydrogénation catalytique, a amené en dernier lieu l'homme de l'art à préconiser à nouveau une voie d'obtention de tagatose entièrement enzymatique à partir de galactose comme il résulte de l'article très récemment publié par D. HALTRICH et al dans « Annals of the New York Academy of Sciences, 864, p 295-299, Dec. 13. 1998 ».

Dans ce document, les auteurs obtiennent du tagatose essentiellement exempt d'aldoses (notamment de galactose et de talose) par la mise en oeuvre d'une pyranose oxydase soluble puis d'une aldose (xylose) réductase (« ALR »). Il apparaît que la galactosone, obtenue avec un rendement de 85 % lors de la première étape, est ensuite entièrement convertie et ce, uniquement en tagatose, lors de l'étape subséquente de réduction enzymatique. Cette étape présente cependant le désavantage d'être complexe à mettre en oeuvre et notamment de rendre obligatoires un contrôle très strict des conditions de milieu pour ne pas désactiver ou déstabiliser l'ALR, ainsi que la régénération permanente du co-facteur (NAD+) nécessaire à cette réaction enzymatique.

Il résulte de ce qui précède qu'il n'existe pas, à ce jour, de moyen qui permette, de manière simple et industrialisable, d'obtenir du tagatose à partir de galactosone et ce, avec à la fois un taux de conversion élevé, voire total et une sélectivité relativement élevée, à savoir au moins égale à 80 %.

Et le mérite de la Société Demanderesse est d'avoir trouvé, après de nombreuses recherches et malgré les enseignements de l'article de FREIMUND précité, qu'un tel moyen pouvait consister en un traitement d'hydrogénation catalytique de la galactosone dès lors que ledit traitement était mené dans des conditions sélectionnées, i.e. des conditions particulières liées à la fois à la concentration de la composition de galactosone soumise à hydrogénation et aux conditions de température et de pression utilisées lors de cette hydrogénation.

De manière plus précise, la présente invention a pour objet un procédé de préparation d'une composition de tagatose comprenant une étape au cours de laquelle on soumet une composition de galactosone à un traitement d'hydrogénation en présence d'au moins un catalyseur, caractérisé en ce que ladite composition de galactosone présente une matière sèche au moins égale à 10 % en poids, de préférence comprise entre 20 et 50 % en poids, et en ce que ledit traitement d'hydrogénation est effectué à une température au moins égale à 40°C et à une pression au moins égale à 10 bars.

Par « composition de galactosone », on entend ici toute composition, en particulier toute solution, contenant de la galactosone en tant que seul sucre ou en mélange quelconque avec au moins un autre sucre (de type cétose ou non, non hydrogéné ou déjà hydrogéné) et ce, quel que soit le procédé ayant été mis en oeuvre pour obtenir ladite composition.

Il peut s'agir de compositions à base de galactosone non purifiée telles que celles obtenues et mises en oeuvre dans certaines des variantes de procédé décrites dans les articles de FREIMUND et HALTRICH précités et qui ne contiennent, par exemple, que 80 % ou 85 %, en poids, de galactosone.

Dans le cadre de la présente invention, la Société Demanderesse a également imaginé que l'on pouvait soumettre à un traitement d'hydrogénation catalytique une composition de galactosone constituée :
- soit d'un mélange contenant galactosone et glucosone et pouvant, selon la Demanderesse, être avantageusement obtenu par action de la pyranose 2-oxydase (« P2O ») sur un mélange galactose / glucose tel qu'un hydrolysat de lactose, purifié ou non,
- soit d'un mélange contenant galactosone et fructose et pouvant, selon la Demanderesse, être avantageusement obtenu par action de la P2O sur un mélange galactose / fructose tel qu'un hydrolysat de lactulose, purifié ou non,
- soit d'une composition contenant de la galactosone comme sucre unique ou fortement majoritaire et pouvant, selon la Demanderesse, être avantageusement obtenu par action de la P2O sur du galactose préalablement extrait d'un hydrolysat de lactose ou de lactulose.

Dans le cas où l'on vise à obtenir, de par la mise en oeuvre du procédé selon l'invention, une composition finale dont le seul sucre souhaité est le tagatose, il est conseillé de partir d'une composition de galactosone purifiée telle que celle utilisée, par exemple, dans l'une des variantes de procédé décrites dans l'article précité de FREIMUND.

De manière avantageuse, la composition de galactosone introduite dans l'enceinte d'hydrogénation catalytique présente, comme indiqué, une matière sèche comprise entre 20 et 50 % en poids. De telles matières sèches sont largement supérieures à celles résultant des exemples décrits dans l'article précité de PREIMUND et qui sont de l'ordre de 8,3 % et 1,8 %.

Selon une autre variante préférentielle du procédé revendiqué, le traitement d'hydrogénation catalytique auquel on soumet ladite composition est effectué :
- à une température au moins égale à 45°C, de préférence comprise entre 50 et 150°C, et
- à une pression au moins égale à 20 bars, de préférence comprise entre 30 et 150 bars.

Tout catalyseur en présence duquel se fait la réaction d'hydrogénation est notamment choisi dans le groupe constitué du palladium, du nickel, du ruthénium, du platine, du rhodium, du cobalt, du cuivre, du zinc, du chrome, du manganèse et du tungstène.

Il peut s'agir notamment de palladium ou de nickel (sous forme Ni de Raney).

La Société Demanderesse a notamment constaté, comme il sera exemplifié ci-après, qu'il était maintenant possible et ce, contrairement aux enseignements de l'article de FREIMUND précité, de convertir de la galactosone en tagatose avec une sélectivité largement supérieure à 76 %, en l'occurrence avec une sélectivité d'au moins 86 % environ, en utilisant un catalyseur constitué de palladium sur charbon.

Dans le procédé conforme à l'invention, le catalyseur est généralement imprégné ou co-échangé sur un support inerte, de préférence choisi dans le groupe constitué du charbon actif, de la tourbe, des zéolites, des aluminosilicates, du dioxyde de titane. De préférence il est constitué de charbon actif.

Le rapport pondéral catalyseur / support inerte est établi avantageusement à une valeur comprise entre 1 et 5 %, de préférence de l'ordre de 5 % comme exemplifié ci-après.

Il est également possible de mettre en oeuvre un catalyseur comportant un agent promoteur. Cet agent promoteur peut être choisi dans le groupe constitué du titane, du molybdène et du platine.

Dans un mode de réalisation du procédé conforme à l'invention, on choisit de conduire l'hydrogénation en mode continu sur lit fixe de catalyseur.

Le catalyseur est alors disposé dans un lit fixe sous la forme d'un empilement compact de particules, le tout placé sur des grilles de soutien dans un réacteur d'hydrogénation. On choisit avantageusement un réacteur à ruissellement.

Au sens de l'invention, on entend par « réacteur à ruissellement », un réacteur d'hydrogénation dans lequel une phase liquide contenant le produit à hydrogéner et une phase gaz circulent de manière co-courante ou contre-courante, de préférence de manière co-courante, de haut en bas dans un lit fixe de particules de catalyseur où s'effectue la réaction d'hydrogénation.

Les débits d'écoulement de ces deux phases sont réglés pour permettre au liquide de ruisseler sur lesdites particules de catalyseur, et assurer le meilleur contact entre les deux phases liquide et gazeuse d'une part, et la phase solide du catalyseur d'autre part.

Dans un mode de réalisation du procédé conforme à l'invention, on choisit de préparer un lit fixe constitué de 200 1 de catalyseur commercial, un débit d'alimentation de la solution de galactosone d'une matière sèche comprise entre 10 et 50 % en poids, à une valeur comprise entre 150 et 250 kg/h, et une quantité d'hydrogène introduite dans ledit réacteur à ruissellement de l'ordre de cinq à quinze fois la stoechiométrie de la réaction.

Les pression et température d'hydrogénation mises en oeuvre sont alors avantageusement choisies à des valeurs respectives de l'ordre de 30 à 120 bars, par exemple 100 bars, et de 100 à 150°C, par exemple 120°C.

Dans un mode préférentiel de réalisation du procédé conforme à l'invention, on choisit de mettre en oeuvre un procédé continu d'hydrogénation de la galactosone dans une succession de lits fixes de catalyseur disposés en série et en au moins deux zones de réaction.

L'hydrogénation est alors avantageusement conduite dans une première zone de réaction, constituée d'au moins un lit fixe de catalyseur, de manière à obtenir une sélectivité relativement élevée en tagatose, puis dans une seconde zone de réaction, constituée d'au moins un lit fixe de catalyseur, où l'hydrogénation est conduite de manière à obtenir, à sélectivité constante voire améliorée, une conversion totale en tagatose.

On peut mettre en oeuvre des catalyseurs de nature différente dans chacune des zones de réaction. Cependant on préfère utiliser un catalyseur de même nature dans ces deux zones.

Dans la première zone de réaction, les conditions de pression et de température sont réglées de manière à obtenir une sélectivité relativement élevée, de l'ordre d'au moins 80 %. On choisit une pression au moins égale à 50 bars, et une température au moins égale à 100°C, de préférence comprise entre 100 et 150°C, comme exemplifié ci-après.

Dans la seconde zone de réaction, les conditions de pression et de température sont réglées pour obtenir un taux de conversion élevé qui peut être de l'ordre de 100 %. On choisit une pression au plus égale à 150 bars, de préférence comprise entre 50 et 100 bars, et une température au plus égale à 100°C, de préférence comprise entre 50 et 100°C, comme exemplifié ci-après.

Les lits fixes de catalyseur sont avantageusement disposés dans des réacteurs à ruissellement. On choisit, par exemple, de mettre en oeuvre 200 1 de particules de catalyseur commercial, un débit d'alimentation de la solution de galactosone d'une matière sèche comprise entre 10 et 50 % en poids à une valeur comprise entre 200 et 400 kg/h, et une quantité d'hydrogène comprise entre cinq et quinze fois la stoechiométrie de la réaction.

En suite de quoi, on dispose désormais d'un nouveau moyen particulièrement simple et efficace de conversion sélective, en tagatose, de la galactosone contenue dans une composition quelconque.

Comme indiqué précédemment, une telle composition peut avoir été obtenue à partir d'une composition de galactose traitée par une enzyme comme la P20, ladite composition de galactose pouvant elle-même être constituée d'un hydrolysat de lactose ou de lactulose, purifié ou non et notamment débarrassé ou non, respectivement, du glucose ou du fructose qu'il contient.

La présente invention a donc également pour objet un procédé de préparation d'une composition de tagatose comprenant :
a) une étape au cours de laquelle on soumet une composition de galactose à l'action d'une enzyme ayant une activité pyranose 2-oxidasique en vue d'obtenir une composition de galactosone,
b) éventuellement, une ou plusieurs étapes intermédiaires de purification de la composition de galactosone ainsi obtenue, et
c) une étape subséquente au cours de laquelle la composition de galactosone, éventuellement purifiée, est soumise à un traitement d'hydrogénation catalytique tel que décrit ci-avant.

Selon une première variante dudit procédé, la composition de galactose se présente sous la forme d'une composition liquide d'une matière sèche au moins égale à 10 % en poids et contenant essentiellement du galactose comme sucre.

Selon une seconde variante, la composition de galactose se présente sous la forme d'un mélange liquide d'une matière sèche au moins égale à 10 % en poids contenant du galactose et au moins un autre sucre, de préférence choisi dans le groupe comprenant le glucose, le fructose et leurs mélanges.

Lorsque la composition de galactose soumise à l'action de l'enzyme de type P2O consiste en un hydrolysat de lactose ou de lactulose, la composition de galactosone résultante consiste en un mélange contenant, au total, au moins 50 %, de préférence au moins 80 %, en poids, de galactosone et, respectivement, de glucosone ou de fructose.

Selon une autre variante préférentielle, de tels mélanges correspondent à des compositions de tagatose contenant du tagatose (hydrogéné ou non), du sorbitol ainsi que, éventuellement du mannitol et ce, selon un ratio pondéral tagatose (hydrogéné ou non) / sorbitol + mannitol compris entre 10/1 et 1/10.

La Société Demanderesse a en effet montré que les mélanges de tagatose, sorbitol et éventuellement du mannitol dans un rapport 10/1 et 1/10, étaient particulièrement adaptées aux applications édulcorantes et agents de charge par rapport à des compositions ne renfermant que du tagatose, du sorbitol, voire un mélange sorbitol + mannitol.

L'invention a également pour objet un procédé de préparation d'une composition de tagatose par hydrogénation catalytique, à une température au moins égale à 40°C et à une pression au moins égale à 10 bars, d'une composition de galactosone présentant une matière sèche au moins égale à 10%.

Le catalyseur utilisé lors du traitement d'hydrogénation peut notamment consister en du palladium sur charbon. La composition de galactosone soumise à hydrogénation peut contenir ou non d'autres sucres tels que la glucosone ou du fructose.

Cette composition peut notamment être obtenue à partir d'une composition de galactose telle qu'un hydrolysat de lactose ou de lactulose, purifié ou non, ayant été soumis à l'action d'une enzyme à activité pyranose 2-oxydasique.

De tels mélanges, lesquels constituent également de nouveaux produits industriels, peuvent, comme l'ensemble des compositions de tagatose susceptibles d'être obtenues selon tout procédé conforme à l'invention, être avantageusement utilisées pour la préparation de produits alimentaires, pharmaceutiques ou cosmétiques, en particulier en tant qu'édulcorants, agents de charge et/ou agents à activité anti-hyperglycémique.

### Exemple

La réaction d'hydrogénation est effectuée dans deux réacteurs à ruissellement connectés en série avec un échangeur thermique intermédiaire.

Les deux réacteurs renferment chacun un unique lit fixe de catalyseur au palladium sur charbon, dans lequel on fait circuler l'hydrogène et la solution de galactosone de manière co-courante du haut vers le bas desdits réacteurs.

Chaque lit fixe de catalyseur au palladium est constitué d'un empilement compact de grains de catalyseur cylindriques.

Chaque grain de catalyseur est composé de charbon actif de type NORIT RX08, sous la forme d'un cylindre de 0,8 mm de diamètre et de 1 à 5 mm de longueur, contenant 5 % en poids de palladium.

Chaque réacteur contient 200 1 de catalyseur, disposé en lit fixe de 30 cm de diamètre et de l'ordre de 3 m de hauteur.

On alimente simultanément la première zone d'hydrogénation avec une solution de galactosone à 25 % de matière sèche, à un débit de 300 kg/h et de l'hydrogène à raison de 15 kg/h.

La pression de fonctionnement dans le premier réacteur est de 50 bars, et la température est fixée à 120°C.

Dans le second réacteur, la pression et la température sont amenées à 80 bars et 90 °C, respectivement.

A la sortie du premier réacteur, le taux de conversion est de 84,6 % avec une sélectivité de 80,7 %.

A la sortie du second réacteur, la conversion est totale, avec une sélectivité augmentée à une valeur de 86,4 %.

## Revendications

1. Procédé de préparation d'une composition de tagatose comprenant une étape au cours de laquelle on soumet une composition de galactosone à un traitement d'hydrogénation en présence d'au moins un catalyseur, caractérisé en ce que ladite composition de galactosone présente une matière sèche au moins égale à 10 % en poids, de préférence comprise entre 20 et 50 % en poids, et en ce que ledit traitement d'hydrogénation est effectué à une température au moins égale à 40°C et à une pression au moins égale à 10 bars.

2. Procédé selon la revendication 1, caractérisé en ce que le traitement d'hydrogénation est effectué :
- à une température au moins égale à 45°C, de préférence comprise entre 50 et 150°C, et
- à une pression au moins égale à 20 bars, de préférence comprise entre 30 et 150 bars.

3. Procédé selon l'une ou l'autre des revendications 1 et 2, caractérisé en ce que le catalyseur est choisi dans le groupe constitué du palladium, du nickel, du ruthénium, du platine, du rhodium, du cobalt, du cuivre, du zinc, du chrome, du manganèse et du tungstène, et est de préférence du palladium.

4. Procédé selon l'une quelconque des revendications 1 à 3, caractérisé en ce que l'on conduit l'hydrogénation de la galactosone en tagatose en mode continu sur lit(s) fixe(s) de catalyseur.

5. Procédé selon la revendication 4, caractérisé en ce que l'on conduit l'hydrogénation continue de la galactosone en tagatose dans une succession de lits fixes de catalyseur disposés en série qui comprend :
- une première zone d'hydrogénation, constituée d'au moins un lit fixe de catalyseur, où la pression est au moins égale à 50 bars et la température est au moins égale à 100°C, de préférence comprise entre 100 et 150°C, et
- une seconde zone d'hydrogénation, constituée d'au moins un lit fixe de catalyseur, où la pression est au plus égale à 100 bars et la température est au plus égale à 100°C, de préférence comprise entre 50 et 100°C.

6. Procédé de préparation d'une composition de tagatose comprenant :
a) une étape au cours de laquelle on soumet une composition de galactose à l'action d'une enzyme ayant une activité pyranose 2-oxydasique en vue d'obtenir une composition de galactosone,
b) éventuellement, une ou plusieurs étapes intermédiaires de purification de la composition de galactosone ainsi obtenue, et
c) une étape subséquente au cours de laquelle on soumet la composition de galactosone, éventuellement purifiée et présentant une matière sèche au moins égale à 10 % en poids, à un traitement d'hydrogénation effectué en présence d'au moins un catalyseur, à une température au moins égale à 40°C et à une pression au moins égale à 10 bars.

7. Procédé selon la revendication 6, caractérisé en ce que la composition de galactose mise en oeuvre lors de l'étape a) consiste en un hydrolysat de lactose ou en un hydrolysat de lactulose.

8. Procédé selon l'une quelconque des revendications 1 à 7, caractérisé en ce que la composition de galactosone soumise au traitement d'hydrogénation consiste en un mélange contenant, au total, au moins 50 % en poids, de préférence au moins 80 % en poids, de galactosone et de glucosone ou de fructose.

9. Composition de tagatose, caractérisée en ce qu'elle contient du tagatose, du sorbitol ainsi que, éventuellement, du mannitol, le ratio pondéral tagatose/sorbitol + mannitol de ladite composition étant compris entre 10/1 et 1/10.

10. Composition de tagatose selon la revendication 9, caractérisée en ce que tout ou partie du tagatose est hydrogéné.

11. Utilisation d'une composition de tagatose selon l'une ou l'autre des revendications 9 et 10, ou obtenue selon l'une quelconque des revendications 1 à 8, dans la préparation de produits alimentaires, pharmaceutiques ou cosmétiques, en particulier en tant qu'agent édulcorant, agent de charge et/ou agent à activité anti-hyperglycémique.
